(19)

Europäisches
Patentamt

European
Patent Office

Office européen
des brevets

(11)  **EP 4 151 651 A1**

(12)  **EUROPEAN PATENT APPLICATION**

(43) Date of publication:
 **22.03.2023  Bulletin 2023/12**

(51) International Patent Classification (IPC):
 **C07K 16/24** *(2006.01)*

(21) Application number: **22195750.9**

(22) Date of filing: **15.09.2022**

(52) Cooperative Patent Classification (CPC):
 **C07K 16/247; C07K 16/244; C07K 16/248;**
 C07K 2317/21; C07K 2317/565; C07K 2317/567;
 C07K 2317/92

(84) Designated Contracting States:
 **AL AT BE BG CH CY CZ DE DK EE ES FI FR GB
 GR HR HU IE IS IT LI LT LU LV MC MK MT NL NO
 PL PT RO RS SE SI SK SM TR**
 Designated Extension States:
 **BA ME**
 Designated Validation States:
 **KH MA MD TN**

(30) Priority: **15.09.2021  JP 2021150418**

(71) Applicant: **SYSMEX CORPORATION**
 **Kobe-shi**
 **Hyogo 651-0073 (JP)**

(72) Inventors:
 • **EGASHIRA, Yuriko**
  **Kobe-shi, Hyogo, 651-0073 (JP)**

 • **DIXIT, Shivani**
  **Kobe-shi, Hyogo, 651-0073 (JP)**
 • **TANAKA, Kazuki**
  **Kobe-shi, Hyogo, 651-0073 (JP)**
 • **MAETA, Shingo**
  **Kobe-shi, Hyogo, 651-0073 (JP)**

(74) Representative: **De Clercq & Partners**
 **Edgard Gevaertdreef 10a**
 **9830 Sint-Martens-Latem (BE)**

Remarks:
 The complete document including Reference
 Table(s) and the Sequence Listing(s) can be
 downloaded from the EPO website

(54)  **METHOD FOR IMPROVING AFFINITY OF ANTI-CYTOKINE ANTIBODY FOR ANTIGEN,
 METHOD FOR PRODUCING ANTI-CYTOKINE ANTIBODY, AND ANTI-CYTOKINE ANTIBODY**

(57)  Disclosed is a method for improving an affinity of an anti-cytokine antibody for an antigen, the method comprising: in an anti-cytokine antibody whose electrical characteristic of complementarity determining regions (CDRs) is negatively charged, changing at least 3 amino acid residues of light chain framework region 3 to arginine residues or lysine residues, thereby improving an affinity for an antigen as compared with that of an antibody before the at least 3 amino acid residues are changed to arginine residues or lysine residues, wherein the at least 3 amino acid residues comprise residues at least 3 positions selected from the group consisting of positions 63, 65, 67, 70 and 72 of a light chain defined by Chothia method.

**Description**

FIELD OF THE INVENTION

The present invention relates to a method for improving affinity of an anti-cytokine antibody for an antigen. The present invention relates to a method for producing an anti-cytokine antibody. The present invention relates to an anti-cytokine antibody.

BACKGROUND

Methods for controlling affinity of an antibody for an antigen by modifying an amino acid sequence of a framework region (FR) while maintaining the amino acid sequence of complementarity determining regions (CDRs) of the antibody are known in the art. The FR is a region other than CDRs, present in each variable region of the light chain and heavy chain of an antibody. The FR plays the role of a scaffold linking three CDRs and contributes to structural stability of the CDR. Therefore, the amino acid sequence of the FR is highly conserved between antibodies of the same species. Each variable region of the heavy chain and light chain has three CDRs, CDR1, CDR2 and CDR3, and four FRs, FR1, FR2, FR3 and FR4. These are arranged in the order of FR1, CDR1, FR2, CDR2, FR3, CDR3 and FR4, starting from the N-terminal side of the variable region. U.S. Patent Application Publication No. 2018/0179298 describes a method to control the affinity of an antibody for an antigen by changing at least 3 amino acid residues in FR3 of the light chain to charged amino acid residues.

SUMMARY OF THE INVENTION

An object of the present invention is to provide a novel method for improving the affinity of an anti-cytokine antibody for an antigen by modifying amino acid residues of light chain FR, a novel anti-cytokine antibody with improved affinity for an antigen, and a production method thereof.
The present invention provides a method for improving an affinity of an anti-cytokine antibody for an antigen, the method including: in an anti-cytokine antibody of which the electrical characteristic of the CDRs is negatively charged, changing at least 3 amino acid residues of light chain FR3 to arginine residues or lysine residues, thereby improving an affinity for an antigen as compared with that of an antibody before the at least 3 amino acid residues are changed to arginine residues or lysine residues, in which the electrical characteristic of the CDRs is determined by a following formula, and the at least 3 amino acid residues include residues in at least 3 positions selected from the group consisting of positions 63, 65, 67, 70 and 72 of a light chain defined by Chothia method.
In particular embodiments, X = [Number of basic amino acid residues in amino acid sequences of the CDRs contained in one antigen binding site] - [Number of acidic amino acid residues in amino acid sequences of the CDRs contained in one antigen binding site],

    wherein when X is -2 or less, the electrical characteristic of the CDRs is negatively charged,
    when X is -1, 0 or 1, the electrical characteristic of the CDRs is neutral, and
    when X is 2 or more, the electrical characteristic of the CDRs is positively charged.

The present invention provides a method for producing an anti-cytokine antibody with an improved affinity for an antigen, the method including: in an anti-cytokine antibody of which the electrical characteristic of the CDRs is negatively charged, generating an antibody in which at least 3 amino acid residues of light chain FR3 are changed to arginine residues or lysine residues; and recovering the antibody generated in the generating, in which an affinity of the recovered antibody for an antigen is improved as compared with that of an antibody before the at least 3 amino acid residues are changed to arginine residues or lysine residues, the electrical characteristic of the CDRs is determined by the above formula, and the at least 3 amino acid residues include residues at least 3 positions selected from the group consisting of positions 63, 65, 67, 70 and 72 of a light chain defined by Chothia method.
In one embodiment, the anti-cytokine antibody is an anti-interleukin antibody.
In one embodiment, the anti-interleukin antibody is an anti-IL-6 antibody, an anti-IL-8 antibody, an anti-IL-12 antibody, or an anti-IL-4 antibody.
In one embodiment, the at least 3 amino acid residues comprise amino acid residues at positions 63, 65 and 67 of the light chain.
In one embodiment, the at least 3 amino acid residues comprise amino acid residues at positions 63, 65, 67 and 70 of the light chain.
In one embodiment, the at least 3 amino acid residues comprise amino acid residues at positions 63, 65, 67, 70 and 72 of the light chain.

The present invention provides an anti-cytokine antibody in which at least 3 amino acid residues of light chain FR3 are changed to arginine residues or lysine residues, in which the anti-cytokine antibody is an antibody of which the electrical characteristic of the CDRs is negatively charged, whereby the electrical characteristic of the CDRs is determined by the above formula, the at least 3 amino acid residues include residues in at least 3 positions selected from the group consisting of positions 63, 65, 67, 70 and 72 of a light chain defined by Chothia method, and an affinity of the anti-cytokine antibody for an antigen is higher than that of an antibody before the at least 3 amino acid residues are changed to arginine residues or lysine residues.

In one embodiment, the anti-cytokine antibody is an anti-interleukin antibody.

In one embodiment, the anti-interleukin antibody is an anti-IL-6 antibody, an anti-IL-8 antibody, an anti-IL-12 antibody, or an anti-IL-4 antibody.

In one embodiment, wherein the at least 3 amino acid residues comprise amino acid residues at positions 63, 65 and 67 of the light chain.

In one embodiment, the at least 3 amino acid residues comprise amino acid residues at positions 63, 65, 67 and 70 of the light chain.

In one embodiment, the at least 3 amino acid residues comprise amino acid residues at positions 63, 65, 67, 70 and 72 of the light chain.

According to the present invention, an anti-cytokine antibody with improved affinity for an antigen is provided.

BRIEF DESCRIPTION OF THE DRAWINGS

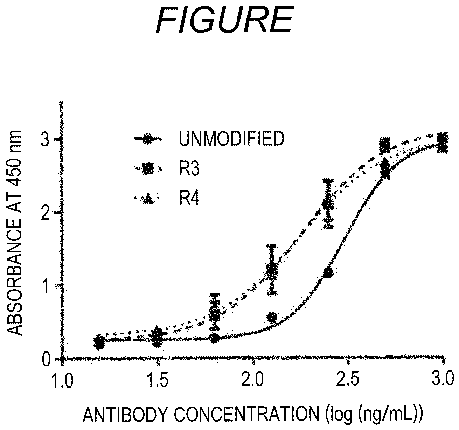

Fig. 1 is a graph showing results of measuring affinity of an anti-interleukin (IL)-4 antibody and variants thereof for an antigen (recombinant human IL-4) by ELISA method.

DETAILED DESCRIPTION OF THE PREFERRED EMBODIMENTS

In the method for improving affinity of an anti-cytokine antibody for an antigen of the present invention (hereinafter, also simply referred to as "method"), in light chain FR3 of an anti-cytokine antibody of which the electrical characteristic of the CDRs is negatively charged, at least 3 amino acid residues (hereinafter, also called "predetermined amino acid residues") including residues in at least 3 positions selected from the group consisting of positions 63, 65, 67, 70 and 72 of the light chain defined by Chothia method are changed to arginine residues or lysine residues. Thereby, affinity for an antigen is improved as compared with that of an antibody before the predetermined amino acid residues are changed to arginine residues or lysine residues.

In the present specification, the "antibody" is a concept including an antibody fragment. In the present invention, the antibody fragment preferably contains a variable region of a light chain. Examples of such an antibody fragment include Fab, Fab', F(ab')2, Fd, Fd', Fv, scFv, domain antibody (dAb), reduced IgG (rIgG), diabody, triabody, and the like.

The "one antigen binding site" is a site composed of one heavy chain variable region and one light chain variable region, and is a site that binds to an antigen in an antibody. The number of antigen binding sites of an antibody varies depending on class and form of the antibody. For example, when an antibody is IgG or F(ab')2, the antibody has two antigen binding sites. When an antibody is Fab, the antibody has one antigen binding site. The "CDRs contained in one antigen binding site" refers to all CDRs present in one heavy chain variable region and one light chain variable region constituting the one antigen binding site. That is, the "CDRs contained in one antigen binding site" is a total of six CDRs, i.e. of CDR1, CDR2 and CDR3 in one heavy chain variable region and CDR1, CDR2 and CDR3 in one light chain variable region.

The antibody to which the method of the present invention is applied is an anti-cytokine antibody of which the electrical characteristic of the CDR is negatively charged. In the present specification, the "electrical characteristic of the CDRs" is determined by a following formula:

$$X = [\text{Number of basic amino acid residues in amino acid sequences of the}$$

$$\text{CDRs contained in one antigen binding site}] - [\text{Number of acidic amino acid residues in}$$

$$\text{amino acid sequences of the CDRs contained in one antigen binding site}]$$

wherein when X is -2 or less, the electrical characteristic of the CDRs is negatively charged,
when X is -1, 0 or 1, the electrical characteristic of the CDRs is neutral, and
when X is 2 or more, the electrical characteristic of the CDRs is positively charged.

As can be seen from the above formula, the electrical characteristic of the CDRs is determined based on the number of acidic amino acid residues and basic amino acid residues in the amino acid sequence of the CDRs contained in one antigen binding site. When X calculated by the above formula is -2 or less, that is, when the number of acidic amino acid residues is larger than the number of basic amino acid residues by 2 or more, in the amino acid sequence of the CDRs contained in one antigen binding site, the electrical characteristic of the CDRs is determined to be negatively charged. In other words, when the total number of acidic amino acid residues contained in the six CDRs of the light chain CDR1, the light chain CDR2, the light chain CDR3, the heavy chain CDR1, the heavy chain CDR2 and the heavy chain CDR3 is larger than the total number of basic amino acid residues by 2 or more, the electrical characteristic of the CDRs is defined to be negatively charged. Here, the acidic amino acid residues are an aspartic acid residue and a glutamic acid residue, and the basic amino acid residues are an arginine residue and a lysine residue. In the present specification, a histidine residue is not included in the basic amino acid residues. For example, in the anti-IL-6 antibody (clone 58-1) of Example 1 described later, the number of acidic amino acid residues in the amino acid sequence of the CDRs contained in one antigen binding site is 6, and the number of basic amino acid residues is 2 (see Table 1). Therefore, the anti-IL-6 antibody is an anti-cytokine antibody whose electrical characteristic of the CDRs is negatively charged.

In the art, a method of numbering the amino acid residues of the CDRs (hereinafter, also referred to as "numbering method") for defining boundary and length of the CDRs is known. When the amino acid residues of the CDRs are numbered by the numbering method, the amino acid residues of the FRs are also numbered. The number assigned to the amino acid residue by the numbering method indicates a position of the amino acid residue in the amino acid sequence of the light chain or the heavy chain. In the present specification, boundaries and lengths of the CDRs and FR3 are defined by Chothia method (see Chothia C. and Lesk AM., Canonical Structures for the Hypervariable Regions of Immunoglobulins., J Mol Biol., vol. 196, pp. 901-917, 1987), which is one of known numbering methods. In the Chothia method, the light chain FR3 is defined as a region consisting of amino acid residues at positions 57 to 88 of the light chain. Hereinafter, when the position of an amino acid residue in the light chain of an antibody is described, the position of the amino acid residue means a position defined by the Chothia method unless otherwise specified.

The term "light chain FR3" means not only FR3 in a full-length light chain, but also FR3 in a portion of the light chain (e.g., light chain variable region). For example, in the case of an antibody fragment that does not contain a full-length light chain and contains a light chain variable region (for example, Fab), the "light chain FR3" refers to FR3 in the light chain variable region in the antibody fragment.

In the light chain FR3, an anti-cytokine antibody before the predetermined amino acid residues are changed to arginine residues or lysine residues is hereinafter also called "original antibody". In the present specification, the "original antibody" means an anti-cytokine antibody having an amino acid sequence before applying the method of the present invention. In the light chain FR3 of the original antibody, changing the predetermined amino acid residues to arginine residues or lysine residues is hereinafter also referred to as "modify" or "modification". The anti-cytokine antibody having an amino acid sequence after applying the method of the present invention to the original antibody is hereinafter also called "modified antibody" or "variant".

The original antibody is an antibody whose electrical characteristic of the CDRs is negatively charged, which specifically binds to a cytokine as an antigen. Since the modification of the present invention is not a modification of the CDRs, the modified antibody specifically binds to a cytokine like the original antibody, and the electrical characteristic of the CDRs is negatively charged. The cytokine is not particularly limited, and examples thereof include interleukins, interferons, chemokines, tumor necrosis factors, cell growth factors, and the like. In a preferred embodiment, the original antibody is an anti-interleukin antibody. The anti-interleukin antibody can be appropriately selected from antibodies that specifically bind to known interleukins. Examples thereof include an anti-IL-6 antibody, an anti-IL-8 antibody, an anti-IL-12 antibody, an anti-IL-4 antibody, and the like.

Since whether or not the electrical characteristic of the CDRs of the original antibody is negatively charged is determined by the amino acid sequence of the CDRs contained in one antigen binding site, the original antibody is preferably an anti-cytokine antibody in which base sequences of genes encoding variable regions of the light chain and the heavy chain are known or the base sequences can be confirmed. Alternatively, the original antibody may be an anti-cytokine antibody whose amino acid sequences of the variable regions of the light chain and the heavy chain are known. Examples of such an antibody include an antibody in which a base sequence of an antibody gene or amino acid sequences of the variable regions are disclosed in a known database, an antibody from which an antibody-producing hybridoma is available, and the like. Examples of the known database include PDB, GeneBank, abYsis, IMGT, and the like.

The original antibody includes not only an antibody having a natural amino acid sequence but also an antibody in which the amino acid sequence is artificially changed by a means other than the method of the present invention. Examples of the antibody in which the amino acid sequence has been artificially changed include an antibody in which an amino acid sequence of the CDRs has been changed, a chimeric antibody, a humanized antibody, a bispecific antibody, a multispecific antibody, a single-chain antibody (scFv) in a chimeric antigen receptor, and the like. In addition to these, in order to artificially manipulate properties of an antibody, a technique of artificially changing an amino acid sequence is known. Such an artificially designed antibody may also be the "original antibody".

When the original antibody is a bispecific antibody or a multispecific antibody, the original antibody preferably has at least one antigen binding site satisfying the following conditions a) and b). The method of the present invention can be applied to the light chain FR3 of the antigen binding site satisfying the conditions among the antigen binding sites of the bispecific antibody and the multispecific antibody. Since the modification of the present invention is not a modification of the CDRs, when the modification is made to a bispecific antibody or a multispecific antibody, the modified antibody has bispecific or multispecific properties similar to those of the original antibody.

In particular embodiments, the original antibody is an antibody, characterized in that: the amino acid sequence of the CDRs contained in one heavy chain variable region and one light chain variable region constituting the antigen binding site, the number of acidic amino acid residues is more than the number of basic amino acid residues by 2 or more. In particular embodiments, the original antibody is an antibody satisfying at least one conditions a) and b):

a) The antigen binding site specifically binds to a cytokine.
b) In the amino acid sequence of the CDRs contained in one heavy chain variable region and one light chain variable region constituting the antigen binding site, the number of acidic amino acid residues is more than the number of basic amino acid residues by 2 or more.

The original antibody may be an antibody derived from any animal, and examples thereof include antibodies derived from human, mouse, rat, hamster, rabbit, goat, horse, chicken, and the like. Class of original antibody may be any of IgG, IgA, IgM, IgD and IgE, and is preferably IgG. The original antibody may be an antibody fragment as long as it contains a variable region of a light chain.

The number of amino acid residues that is modified by the methods of the present invention in the light chain FR3 of the original antibody is, for example, 14, 13, 12, 11, 10, 9, 8, 7, 6 or 5. In one embodiment, the number of amino acid residues that is modified by the methods of the present invention in the light chain FR3 of the original antibody is, for example, 3 or more and 5 or less.

The predetermined amino acid residues that are changed to arginine residues or lysine residues in the light chain FR3 of the original antibody by the methods of the present invention may be three or four amino acid residues selected from the group consisting of positions 63, 65, 67, 70 and 72 of the light chain. Alternatively, the predetermined amino acid residues changed by the methods of the present invention to arginine residues or lysine residues in the light chain FR3 of the original antibody may be amino acid residues at positions 63, 65, 67, 70 and 72 of the light chain.

Preferably, the predetermined amino acid residues changed by the methods of the present invention to arginine residues or lysine residues in the light chain FR3 of the original antibody are any of the following 1) to 3):

1) amino acid residues at positions 63, 65 and 67 of the light chain defined by the Chothia method;
2) amino acid residues at positions 63, 65, 67 and 70 of the light chain defined by the Chothia method; and
3) amino acid residues at positions 63, 65, 67, 70 and 72 of the light chain defined by the Chothia method.

When amino acid residues other than positions 63, 65, 67, 70 and 72 of the light chain are modified in the light chain FR3 of the original antibody, it is preferable to modify amino acid residues excluding a Vernier zone residue and an unexposed residue from the amino acid sequence of the light chain FR3. The term "Vernier zone residue" is an amino acid residue contributing to structural stability of the CDR among the amino acid sequence of FR. The term "unexposed residue" is an amino acid residue that is folded inside the molecule and is not exposed to the surface. It is expected that even if the unexposed residue is modified, the effect of the modification is small or absent. The amino acid residues excluding the Vernier zone residue and the unexposed residue from the amino acid sequence of the light chain FR3 are specifically amino acid residues at positions 57 to 63, 65, 67, 70, 72, 74, 76, 77 and 79 to 81 of the light chain. Therefore, when amino acid residues other than positions 63, 65, 67, 70 and 72 of the light chain are modified, an amino acid residue selected from amino acid residues at positions 57 to 62, 74, 76, 77 and 79 to 81 of the light chain is preferably an arginine residue or a lysine residue.

The amino acid residue in the light chain FR3 of the original antibody before modification is a neutral amino acid residue, an acidic amino acid residue, or a histidine residue. Preferably, the amino acid residue before modification in the light chain FR3 of the original antibody is a neutral amino acid residue or an acidic amino acid residue. The neutral amino acid residue refers to an alanine residue, an asparagine residue, an isoleucine residue, a glycine residue, a glutamine residue, a cysteine residue, a threonine residue, a serine residue, a tyrosine residue, a phenylalanine residue, a proline residue, a valine residue, a methionine residue, a leucine residue, and a tryptophan residue. Accordingly, the original antibody is additionally characterized in that one or more, preferably two or more, more preferably three or more, most preferably all five of the amino acid residues at positions 63, 65, 67, 70 and 72 of the light chain FR3 is not an arginine or lysine residue, and is preferably a neutral amino acid residue.

In the modified antibody obtained by the methods of the present invention, amino acid residues after being modified from amino acid residues of the original antibody may be all arginine residues or all lysine residues. Alternatively, a part of the amino acid residues after being modified from the amino acid residues of the original antibody may be arginine residues, and the rest may be lysine residues.

When one or two of the amino acid residues at positions 63, 65, 67, 70 and 72 of the light chain of the original antibody are arginine residues or lysine residues, the amino acid residues may be left as they are. In this case, among the amino acid residues at positions 63, 65, 67, 70 and 72 of the light chain, at least 3 of the remaining amino acid residues that are not arginine residues and lysine residues may be modified by the methods of the present invention.

Since the CDR is involved in specificity of the antibody, it is preferable that the amino acid sequence of the CDRs is not changed in the method of the present invention. That is, the amino acid sequence of the CDRs of the modified antibody is preferably the same as the amino acid sequence of the CDRs of the original antibody. Naturally, when the amino acid sequence of the CDRs of the modified antibody is the same as the amino acid sequence of the CDRs of the original antibody, the electrical characteristics of the CDRs of these antibodies are also the same.

Examples of means for modifying an amino acid residue in accordance with the methods of the present invention include substitution and insertion of an amino acid residue, and the like. For example, when at least 3 amino acid residues selected from positions 63, 65, 67, 70 and 72 of the light chain of the original antibody are modified by substitution of amino acid residues, the selected amino acid residue is substituted with an arginine residue or a lysine residue. Thereby, the at least 3 amino acid residues change to arginine residues or lysine residues. In modification by insertion of amino acid residues, for example, at least 3 arginine residues or lysine residues are inserted in the amino acid sequence of the original antibody so that the arginine residues or lysine residues are located in at least 3 positions selected from positions 63, 65, 67, 70 and 72 of the light chain of the original antibody. As an example, when positions 63, 65 and 67 of the light chain of the original antibody are changed to arginine residues, arginine residues are inserted between an amino acid residue at position 62 and an amino acid residue at position 63, between the amino acid residue at position 63 and an amino acid residue at position 64, and between the amino acid residue at position 64 and an amino acid residue at position 65, respectively. Thereby, the 3 inserted arginine residues become residues at positions 63, 65 and 67 of the light chain of the modified antibody, respectively.

The original antibody can be modified by known DNA recombination technology, other molecular biological techniques, or the like. Specifically, first, a polynucleotide encoding an amino acid sequence of the original antibody is obtained, and from this polynucleotide, a polynucleotide encoding an amino acid sequence of a modified antibody is prepared by modifying the relevant nucleotides by recombination technology. Then, a modified antibody is generated by a protein expression system using the prepared polynucleotide. The protein expression system may be an expression system using a host cell or a cell-free protein synthesis system. Examples of the host cell include mammalian cells, insect cells, E. coli, yeast, and the like. Examples of the cell-free protein synthesis system include a wheat germ-derived synthesis system, an E. coli-derived synthesis system, a reconfiguration type cell-free protein synthesis system, and the like.

For example, when there is a hybridoma that produces the original antibody, a modified antibody can be obtained as follows. First, RNA extracted from the hybridoma is used to synthesize a polynucleotide encoding the light chain and a polynucleotide encoding the heavy chain of the original antibody, by a reverse transcription reaction and a RACE (rapid amplification of cDNA ends) method. Next, the polynucleotide encoding the light chain as a template is amplified by PCR using a primer for modifying at least 3 amino acid residues of light chain FR3 to obtain a polynucleotide encoding the light chain in which FR3 has been modified. The obtained polynucleotide encoding the modified light chain and the polynucleotide encoding the heavy chain of the original antibody are incorporated into a known expression vector to obtain an expression vector containing a polynucleotide encoding a modified antibody. By transforming or transfecting the obtained expression vector into an appropriate host cell, an antibody with improved affinity is generated. A modified antibody can be obtained by recovering the generated antibody from the host cell.

In the methods of the present invention, the polynucleotide encoding the light chain in which FR3 is modified and the polynucleotide encoding the heavy chain may be incorporated into one expression vector or may be separately incorporated into two expression vectors. The type of expression vector is not particularly limited, and can be determined according to the host cell. Examples thereof include expression vectors for mammalian cells, expression vectors for insect cells, expression vectors for E. coli, expression vectors for yeast, and the like.

When a modified antibody is obtained by a cell-free protein synthesis system, a polynucleotide encoding the light chain in which FR3 has been modified and a polynucleotide encoding the heavy chain of the original antibody are added to the cell-free protein synthesis system and incubated under appropriate conditions, thereby a modified antibody can be obtained.

When producing a modified antibody which is a single chain antibody (scFv), as shown in, for example, PCT International Application Publication No. 2013/084371 A1, RNA extracted from the hybridoma that produces the original antibody may be used to synthesize a polynucleotide encoding a light chain variable region and a polynucleotide encoding a heavy chain variable region by a reverse transcription reaction and PCR. These polynucleotides are ligated by overlap extension PCR or the like to obtain a polynucleotide encoding the original antibody which is scFv. The obtained polynucleotide is amplified by PCR using a primer for modifying at least 3 amino acid residues of light chain FR3 to obtain a polynucleotide encoding scFv in which light chain FR3 has been modified. The obtained polynucleotide is incorporated into a known expression vector to obtain an expression vector containing a polynucleotide encoding a modified antibody which is scFv. By transforming or transfecting the obtained expression vector into an appropriate host cell, a modified antibody which is scFv can be obtained.

When there is no hybridoma that produces the original antibody, an antibody-producing hybridoma may be prepared by known methods such as those described in, for example, Kohler and Milstein, Nature, vol.256, pp.495-497, 1975. Alternatively, RNA obtained from the spleen of an animal such as a mouse immunized with a cytokine peptide as an antigen may be used. When using the RNA obtained from the spleen, for example, as shown in Fukunaga A. and Tsumoto K., Protein Eng. Des. Sel. 2013, vol.26, pp.773-780, a polynucleotide encoding scFv having a desired affinity as an original antibody may be selected by a phage display method or the like, from the obtained polynucleotides encoding scFv. Alternatively, an amino acid sequence of a desired anti-cytokine antibody or a base sequence of an antibody gene may be acquired from a known database, and gene synthesis may be performed based on these sequences to obtain an antibody gene.

The affinity of the modified antibody for an antigen may be evaluated by a kinetic parameter in an antigen-antibody reaction or may be evaluated by an immunological measurement method such as an ELISA method. Examples of the kinetic parameter include dissociation constant ($K_D$), binding rate constant ($k_{on}$), and dissociation rate constant ($k_{off}$). Among them, $K_D$ is preferable. Examples of an index of affinity by immunological measurement include a 50% effective concentration (EC50). The kinetic parameter in an antigen-antibody reaction can be obtained by surface plasmon resonance (SPR) technology or the like. Based on the value of $K_D$ or EC50, the affinity of the modified antibody for an antigen may be compared with the affinity of the original antibody for an antigen. The value of $K_D$ in the antigen-antibody reaction of the modified antibody is, for example, about 1/2, about 1/3, about 1/4, about 1/5, about 1/6, about 1/7, about 1/8, about 1/9, about 1/10, about 1/20, about 1/30, about 1/40, about 1/50, about 1/100 or about 1/1000, as compared with that of the original antibody.

The modified antibody can be used, for example, as an active ingredient for various tests and studies, diagnostic agents, or therapeutic agents. The modified antibody may additionally be modified with a known substance such as a fluorescent dye, an enzyme, a radioisotope, biotin, or an anticancer agent.

The present invention also provides a method for producing a modified anti-cytokine antibody. In the method for producing an anti-cytokine antibody with an improved affinity for an antigen of the present invention (hereinafter, also referred to as "production method"), first, in an anti-cytokine antibody whose electrical characteristic of the CDRs is negatively charged, an antibody in which at least 3 amino acid residues of the light chain FR3 are changed to arginine residues or lysine residues is generated. Specifically, similarly to the method of the present invention, in the original antibody, at least 3 amino acid residues including residues at least 3 positions selected from the group consisting of positions 63, 65, 67, 70 and 72 of the light chain defined by the Chothia method are changed to arginine residues or lysine residues.

In the production method of the present invention, the antibody generated by modifying the at least 3 amino acid residues has improved affinity for an antigen as compared with the original antibody. Details of the modification of the original antibody and the amino acid residues are the same as those described for the method of the present invention. In one embodiment, at least 3 amino acid residues to be changed to arginine residues or lysine residues in the light chain FR3 of the original antibody may be, for example, any of 1) to 3) described above.

In the production method of the present invention, the modified antibody can be generated by known DNA recombination technology, other molecular biological techniques, and the like. For example, first, a polynucleotide encoding an amino acid sequence of the modified antibody is prepared from a polynucleotide encoding an amino acid sequence of the original antibody. Then, a modified antibody is generated by a protein expression system using the prepared polynucleotide. Details of the preparation of the polynucleotide encoding an amino acid sequence of the modified antibody and the generation of the modified antibody by the protein expression system are the same as those described for the method of the present invention. In particular embodiments, the production methods of the present invention involve providing an original anti-cytokine antibody (such as having the features described herein above). In particular embodiments, the method comprises the step of providing a hybridoma that produces said anti-cytokine original antibody; in further embodiments the production methods of the present invention comprise the step of preparing an hybridoma producing an antibody directed against a cytokine by known methods such as those described above and obtaining the original anti-cytokine antibody produced by said hybridoma.

Subsequently, in the production method of the present invention, the modified antibody can be obtained by recovering the generated antibody. A method of recovering an antibody from the protein expression system is known per se. For example, when a modified antibody is generated in a host cell, the host cell may be dissolved in a solution containing an appropriate solubilizer to liberate the modified antibody in the solution. When the host cell secretes the generated modified antibody from the inside of the cell into a medium, a culture supernatant may be recovered. In a cell-free protein synthesis system, the synthesized modified antibody is contained in a reaction solution. The modified antibody liberated in a liquid can be recovered by a known method such as affinity chromatography. For example, when the generated modified antibody is IgG, the modified antibody can be recovered by affinity chromatography using protein A or G. If necessary, the recovered modified antibody may be purified by a method known in the art such as gel filtration.

The affinity of the antibody obtained by the production method of the present invention for an antigen may be evaluated by a kinetic parameter in an antigen-antibody reaction or may be evaluated by an immunological measurement method such as an ELISA method. As indexes of affinity of the antibody for an antigen, for example, $K_D$ and EC50 may be used. Also, based on the value of $K_D$ or EC50, the affinity of the antibody obtained by the production method of the present invention for an antigen may be compared with the affinity of the original antibody for an antigen.

The present invention also provides a modified anti-cytokine antibody also in some instances referred to herein as the "antibody of the (present) invention". The modified anti-cytokine antibody of the present invention is an anti-cytokine antibody in which at least 3 amino acid residues of the light chain FR3 are changed to arginine residues or lysine residues. Specifically, the anti-cytokine antibody of the present invention is an anti-cytokine antibody in which at least 3 amino acid residues including residues at least 3 positions selected from the group consisting of positions 63, 65, 67, 70 and 72 of the light chain defined by the Chothia method are changed to arginine residues or lysine residues. The antibody of the present invention can be obtained by the production method of the present invention. In particular embodiments, said modified antibody is an antibody that is not produced by a hybridoma.

The antibody of the present invention is characterized in that the affinity for an antigen is higher than that of an antibody before the at least 3 amino acid residues are changed to arginine residues or lysine residues. The antibody of which the affinity is to be compared is the same as the "original antibody" described above. The affinity of the antibody of the present invention for an antigen may be evaluated by a kinetic parameter in an antigen-antibody reaction or may be evaluated by an immunological measurement method such as an ELISA method. As indexes of affinity of the antibody for an antigen, for example, $K_D$ and EC50 may be used. Also, based on the value of $K_D$ or EC50, the affinity of the antibody of the present invention for an antigen may be compared with the affinity of the original antibody for an antigen. The value of $K_D$ in the antigen-antibody reaction of the antibody of the present invention is, for example, about 1/2, about 1/3, about 1/4, about 1/5, about 1/6, about 1/7, about 1/8, about 1/9, about 1/10, about 1/20, about 1/30, about 1/40, about 1/50, about 1/100 or about 1/1000, as compared with that of the original antibody.

The antibody of the present invention is characterized in that the electrical characteristic of the CDRs is negatively charged. That is, the antibody of the present embodiment is an antibody in which the number of acidic amino acid residues is more than the number of basic amino acid residues by 2 or more, in the amino acid sequence of the CDRs contained in one antigen binding site. Details of the CDRs contained in one antigen binding site and the electrical characteristic of the CDRs are the same as those described for the method of the present invention.

The antibody of the present invention specifically binds to a cytokine as an antigen. The cytokine is not particularly limited, and examples thereof include interleukins, interferons, chemokines, tumor necrosis factors, cell growth factors, and the like. Preferably, the antibody of the present invention is an anti-interleukin antibody. The type of interleukin is not particularly limited. The antibody of the present invention may be, for example, an anti-IL-6 antibody, an anti-IL-8 antibody, an anti-IL-12 antibody, an anti-IL-4 antibody, or the like.

The antibody of the present invention may be an antibody derived from any animal, and examples thereof include antibodies derived from human, mouse, rat, hamster, rabbit, goat, horse, chicken, and the like. Class of the antibody of the present invention may be any of IgG, IgA, IgM, IgD and IgE, and is preferably IgG. The antibody of the present invention may be an antibody fragment as long as it contains a variable region of a light chain. Examples of such an antibody fragment include Fab, Fab', F(ab')2, Fd, Fd', Fv, scFv, dAb, rIgG, diabody, triabody, and the like.

The antibody of the present invention may be a bispecific antibody or a multispecific antibody having an antigen binding site that specifically binds to a cytokine. In this case, the antigen binding site that specifically binds to a cytokine in the antibody of the present invention preferably satisfies the following conditions (a) and (b):

(a) at least 3 amino acid residues including residues at least 3 positions selected from the group consisting of positions 63, 65, 67, 70 and 72 of the light chain defined by the Chothia method at the antigen binding site are changed to arginine residues or lysine residues; and
(b) in the amino acid sequence of the CDRs contained in one heavy chain variable region and one light chain variable region constituting the antigen binding site, the number of acidic amino acid residues is more than the number of basic amino acid residues by 2 or more.

In the antibody of the present invention, 3 or 4 amino acid residues selected from the group consisting of positions 63, 65, 67, 70 and 72 of the light chain may be changed to arginine residues or lysine residues. Alternatively, in the antibody of the present invention, the amino acid residues at positions 63, 65, 67, 70 and 72 of the light chain may be changed to arginine residues or lysine residues.

In the light chain FR3 of the antibody of the present invention, positions that are arginine residues or lysine residues are

any one of the following (1) to (3):

(1) positions 63, 65 and 67 of the light chain defined by the Chothia method;
(2) positions 63, 65, 67 and 70 of the light chain defined by the Chothia method; and
(3) positions 63, 65, 67, 70 and 72 of the light chain defined by the Chothia method.

In the antibody of the present invention, amino acid residues other than positions 63, 65, 67, 70 and 72 of the light chain, for example, amino acid residues selected from positions 57 to 62, 74, 76, 77 and 79 to 81 of the light chain may be arginine residues or lysine residues. As described above, positions 57 to 62, 74, 76, 77 and 79 to 81 of the light chain are positions of amino acid residues excluding the Vernier zone residue and the unexposed residue from the amino acid sequence of the light chain FR3.

Hereinbelow, the present invention will be described in detail by examples, but the present invention is not limited to these examples.

EXAMPLES

Example 1: Preparation of anti-cytokine antibody in which amino acid residue of light chain FR3 is modified

Antibody genes of an anti-IL-6 antibody, an anti-IL-8 antibody, an anti-IL-12 antibody and an anti-IL-4 antibody as original antibodies were each acquired, and variants of each antibody were prepared. Then, affinity of the original antibodies and the variants for an antigen was measured.

(1) Acquisition of genes of anti-cytokine antibodies

(1.1) Acquisition of genes of anti-IL-6 antibody, anti-IL-8 antibody, and anti-IL-12 antibody

Cells were recovered from each culture of two hybridomas producing mouse anti-human IL-6 antibody (clones 58-1 and 44-15), two hybridomas producing mouse anti-human IL-8 antibody (clones 166-1 and 146-1), and a hybridoma producing mouse anti-human IL-12 antibody (clone 22-4). Pellets of cells (about $1 \times 10^7$ cells) were lysed with 1 mL of ISOGEN (NIPPON GENE CO., LTD.), and total RNA was extracted according to the instruction for use. The obtained total RNA and antisense oligo DNA (GTAAGCTCCCTAATGTGCTG: SEQ ID NO: 1) were mixed, heated at 72°C for 10 minutes, then ice-cooled, and annealed. RNaseH (NEB, Inc.) was added to the resulting mixture, and RNaseH treatment was performed at 37°C for 90 minutes to digest a κ chain pseudogene. The mixture after RNaseH treatment was purified using RNeasy MinElute Cleanup kit (QIAGEN).

cDNA Synthesis was performed using the purified RNA sample and SMARTer (registered trademark) RACE 5'/3' kit (Clontech) according to the package insert of the kit. In addition, a 5'RACE reaction was performed using the obtained cDNA as a template. The obtained 5'RACE product was subjected to 1% agarose gel electrophoresis, and purified using a QIAquick Gel Extraction kit (QIAGEN). The resulting 5'RACE product, TaKaRa Ex Taq (registered trademark) (Takara Bio Inc.), 10x Ex Taq (registered trademark) buffer and 2.5 mM dATP were mixed and reacted at 72°C for 60 minutes to add deoxyriboadenosine (dA) to the 3' end of the 5'RACE product. The obtained dA addition product was purified using a QIAquick PCR Purification kit (QIAGEN).

The purified dA addition product was cloned into a pMD20 vector (Takara Bio Inc.). Competent cell DH5α strain was transformed with the obtained plasmid DNA by a conventional method, and applied to a carbenicillin-containing LB agar medium. After the agar medium was incubated at 37°C for 16 hours, single colonies on the agar medium were selected by conventional colony PCR. E. coli of the selected colonies was shake-cultured in a carbenicillin-containing LB liquid medium (2 mL) at 37°C for 16 hours. Plasmid DNA was extracted from the recovered E. coli using the QIAprep Spin Miniprep kit (QIAGEN). Specific operations were carried out according to the package insert of the kit. Sequence analysis was performed on the obtained plasmid DNA using an M13 for primer and an M13 rev primer. Base sequences of these primers were as follows. An antibody gene was cut out from the obtained plasmid DNA by a predetermined restriction enzyme, and inserted into pcDNA3.4 to obtain plasmid DNA for mammalian cell expression.

M13 for: GGTTTTCCCAGTCACGA (SEQ ID NO: 2)
M13 rev: AGGAAACAGCTATGACCATG (SEQ ID NO: 3)

(1.2) Acquisition of gene of anti-IL-4 antibody

Based on information of an amino acid sequence of human anti-IL-4 antibody published in PDB (PDB: 5FHX), a gene of anti-IL-4 antibody was synthesized and inserted into pcDNA3.4. Sequence analysis was performed on the obtained plasmid DNA to acquire plasmid DNA for mammalian cell expression containing the gene of anti-IL-4 antibody (IgG).

(1.3) Amino acid sequence of each gene of anti-cytokine antibody

The amino acid sequences of the heavy chain and light chain of each antibody (Fab) were determined based on the base sequence of each gene of the anti-IL-6 antibody, the anti-IL-8 antibody and the anti-IL-12 antibody acquired from the hybridoma described above. In addition, the amino acid sequence of a known anti-IL-4 antibody was acquired. These amino acid sequences were as follows. In the amino acid sequence of the light chain of each antibody, underlined residues are amino acid residues at positions 63, 65, 67, 70 and 72 of the light chain defined by the Chothia method. Also, the amino acid sequences of the CDRs defined by the Chothia method of each antibody are shown in Tables 1 to 6. As can be seen from Tables 1 to 6, in all the antibodies, the number of acidic amino acid residues was more than the number of basic amino acid residues by 2 or more, in the amino acid sequence of the CDRs contained in one antigen binding site. Therefore, all the antibodies were anti-cytokine antibodies whose electrical characteristic of the CDRs was negatively charged.

· Amino acid sequence of heavy chain of anti-IL-6 antibody (clone 58-1)

EVQLQQSGPELVKPGASVKMSCKASGYTFTSYVMHWVKQKPGQGLE

WIGYINPYNDGTKYNEKFKGKATLTSDKSSSTAYMELSSLTSEDSAVYYCARE

GYGNLERDCWGQGTSVTVSSASTKGPSVFPLAPSSKSTSGGTAALGCLVKDYF

PEPVTVSWNSGALTSGVHTFPAVLQSSGLYSLSSVVTVPSSSLGTQTYICNVNH

KPSNTKVDKKVEPKSCDKT    (SEQ ID NO: 4)

· Amino acid sequence of light chain of anti-IL-6 antibody (clone 58-1)

DIVLTQSPASLAVSLGQRATISCRASESVDGFGISFMNWFQQKPGQPPK

LLIYVASNQGSGVPARFSGSGSGTDFSLNIHPMEEDDSAMYFCQQSKEVPWTFG

GGTKLEIKR    (SEQ ID NO: 5)

[Table 1]

| · Amino acid sequence of the CDRs of anti-IL-6 antibody (clone 58-1) (In the table, underlined portion indicates an acidic amino acid residue, and boldface indicates a basic amino acid residue) | | | |
| --- | --- | --- | --- |
| | **CDR1** | **CDR2** | **CDR3** |
| Heavy chain | **GYTFTSY** (SEQ ID NO: 6) | **PYND** (SEQ ID NO: 7) | **GYGNLERD** (SEQ ID NO: 8) |
| Light chain | **SESVDGFGISF** (SEQ ID NO: 9) | **VAS** | **SKEVPW** (SEQ ID NO: 10) |

· Amino acid sequence of heavy chain of anti-IL-6 antibody (clone 44-15)

DVQLQESGPGLVKPSQTVSLTCTVTGISITTGNYRWSWIRQFPGNKLEW
IGYIYYSGAITYNPSLTSRTTITRDTSMNQFFLEMISLTAEDTATYYCARDRYDY
AVDFWGQGTSVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTK
VDKKVEPKSCDKT     (SEQ ID NO: 11)

· Amino acid sequence of light chain of anti-IL-6 antibody (clone 44-15)

DIQMTQSPASLSASVGETVTITCRSSENIYSFLTWFQQRQGKSPHLLVY
YANTLAEGVPSRFSGSGSGTQFSLQINSLQPEDFGTYYCQHHYGTPWTFGGGTR
LEIKR  (SEQ ID NO: 12)

[Table 2]

| · Amino acid sequence of the CDRs of anti-IL-6 antibody (clone 44-15) (In the table, underlined portion indicates an acidic amino acid residue, and boldface indicates a basic amino acid residue | | | |
|---|---|---|---|
| | CDR1 | CDR2 | CDR3 |
| Heavy chain | **GISITTGNY** (SEQ ID NO: 13) | **YSG** | **RYDYAVD** (SEQ ID NO: 14) |
| Light chain | **SENISF** (SEQ ID NO: 15) | YAN | **HYGTPW** (SEQ ID NO: 16) |

· Amino acid sequence of heavy chain of anti-IL-8 antibody (clone 166-1)

EVKLVESGGGLVKPGGSLKLSCAASGFTFNNYAMSWVRQTPEKRLEW
VASISSGGNTYYPDSVKGRFTLSRDNARNILYLQMSRLRSEDTAMYYCARDKL
RLPNWYFDVWGAGTTVTVSSASTKGPSVFPLAPSSKSTSGGTAALGCLVKDYF
PEPVTVSWNSGALTSGVHTFPAVLQSSGLYSLSSVVTVPSSSLGTQTYICNVNH
KPSNTKVDKKVEPKSCDKT     (SEQ ID NO: 17)

· Amino acid sequence of light chain of anti-IL-8 antibody (clone 166-1)

DIVLTQSPPSLAVSLGQRATISCKASQSVDYDGDSYMNWYQQKPGQPP
KVLIYGASNLESGIPARFSGSGSGTDFTLNIYPVEEEDAATYYCQQSNEDPPTFG
GGTKLEIKR     (SEQ ID NO: 18)

[Table 3]

| · Amino acid sequence of the CDRs of anti-IL-8 antibody (clone 166-1) (In the table, underlined portion indicates an acidic amino acid residue, and boldface indicates a basic amino acid residue) | | | |
|---|---|---|---|
| | **CDR1** | **CDR2** | **CDR3** |
| Heavy chain | **GFTFNNY** (SEQ ID NO: 19) | **SGG** | **SGGKLRLPNWYFD** (SEQ ID NO: 20) |
| Light chain | **SQSVDYDGDSY** (SEQ ID NO: 21) | **GAS** | **SNEDPP** (SEQ ID NO: 22) |

· Amino acid sequence of heavy chain of anti-IL-8 antibody (clone 146-1)

EVQLVESGGGLVKPGGSLKLSCAASGFTFSSYAMSWVRQTPEKRLEW

VATISNGGSYTYYPDSLKGRFTISRDNAKNTLYLQMSSLRSEDTAIYYCARGITG

GVYFDYWGQGTTLTVSSASTKGPSVFPLAPSSKSTSGGTAALGCLVKDYFPEPV

TVSWNSGALTSGVHTFPAVLQSSGLYSLSSVVTVPSSSLGTQTYICNVNHKPSN

TKVDKKVEPKSCDKT  (SEQ ID NO: 23)

· Amino acid sequence of light chain of anti-IL-8 antibody (clone 146-1)

DIQMTQSPASLSASVGETVTITCRASENIYSYLAWYQQKQGKSPHLLVY

DAETLAEGVPSRFSGSGSGTQFSLKINSLQPEDFGSYYCQHHYGTPWTFGGGTK

LEIKR  (SEQ ID NO: 24)

[Table 4]

| · Amino acid sequence of the CDRs of anti-IL-8 antibody (clone 146-1) (In the table, underlined portion indicates an acidic amino acid residue) | | | |
|---|---|---|---|
| | **CDR1** | **CDR2** | **CDR3** |
| Heavy chain | **GFTFSSY** (SEQ ID NO: 25) | **NGGS** (SEQ ID NO: 26) | **ITGGVYFD** (SEQ ID NO: 27) |
| Light chain | **SENIYSY** (SEQ ID NO: 28) | **DAE** | **HYGTPW** (SEQ ID NO: 29) |

· Amino acid sequence of heavy chain of anti-IL-12 antibody (clone 22-4)

EVQLQESGPSLVKPSQTLSLTCSVTGDSITSGYWNWIRKFPGNKLEYM
GYISYSGSTYYNPSLKSRISITRDTSKNQNYLQLNSVTTEDTATYYCARRDTTVV
VPYYFDYWGQGTTLTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPS
NTKVDKKVEPKSCDKT        (SEQ ID NO: 30)

· Amino acid sequence of light chain of anti-IL-12 antibody (clone 22-4)

DIVMTQSHKFMSTSVGDRVSITCKASQDVGTAVAWYQQKPGQSPKLLI
YWASTRHTGVPGRFTGSGSGTDFTLTINNVQSEDLADYFCQQYSSYPLTFGAGT
KLELKR        (SEQ ID NO: 31)

[Table 5]

· Amino acid sequence of the CDRs of anti-IL-12 antibody (clone 22-4) (In the table, underlined portion indicates an acidic amino acid residue)

|  | CDR1 | CDR2 | CDR3 |
|---|---|---|---|
| Heavy chain | **GDSITSG** (SEQ ID NO: 32) | **YSG** | **DTTVVVPYYFD** (SEQ ID NO: 33) |
| Light chain | **SQDVGTA** (SEQ ID NO: 34) | **WAS** | **YSSYPL** (SEQ ID NO: 35) |

· Amino acid sequence of heavy chain of anti-IL-4 antibody

AVQLQQSGPELVKPGASVKISCKASGYSFTSYWIHWIKQRPGQGLEWI
GMIDPSDGETRLNQRFQGRATLTVDESTSTAYMQLRSPTSEDSAVYYCTRLKE
YGNYDSFYFDVWGAGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPE
VTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRW
QQGNVFSCSVMHEALHNHYTQKSLSLSPGK   (SEQ ID NO: 36)

· Amino acid sequence of light chain of anti-IL-4 antibody

DIQMTQSPASLSVSVGDTITLTCHASQNIDVWLSWFQQKPGNIPKLLIY

KASNLHTGVPSRFSGSGSGTGFTLTISSLQPEDIATYYCQQAHSYPFTFGGGTKL

EIKRTVAAPSVFIFPPSDEQLKSGTASVVCLLNNFYPREAKVQWKVDNALQSGN

SQESVTEQDSKDSTYSLSSTLTLSKADYEKHKVYACEVTHQGLSSPVTMSFNRG

EC      (SEQ ID NO: 37)

[Table 6]

| · Amino acid sequence of the CDRs of anti-IL-4 antibody (In the table, underlined portion indicates an acidic amino acid residue, and boldface indicates a basic amino acid residue) | | | |
|---|---|---|---|
| | CDR1 | CDR2 | CDR3 |
| Heavy chain | GYSFTSY (SEQ ID NO: 38) | PSBG (SEQ ID NO: 39) | KEYGNYDSFYFD (SEQ ID NO: 40) |
| Light chain | SQNIDVW (SEQ ID NO: 41) | KAS | AHSYPF (SEQ ID NO: 42) |

(2) Acquisition of genes of variants of anti-cytokine antibodies

[Reagents]

QIAprep Spin Miniprep kit (QIAGEN)
PrimeSTAR (registered trademark) Max DNA Polymerase (Takara Bio Inc.)
Ligation high ver.2 (TOYOBO CO., LTD.)
T4 Polynucleotide Kinase (TOYOBO CO., LTD.)
Dpn I (TOYOBO CO., LTD.)
Competent high DH5α (TOYOBO CO., LTD.)

(2.1) Primer design and PCR

Based on a base sequence of the wild-type anti-cytokine antibody gene in each of the acquired plasmid DNAs, a primer set for obtaining a polynucleotide encoding a heavy chain and a primer set for obtaining a polynucleotide encoding a light chain in which the following 3, 4 or 5 amino acid residues in FR3 were substituted with arginine residues were designed.

- amino acid residues at positions 63, 65 and 67 of the light chain defined by the Chothia method;
- amino acid residues at positions 63, 65, 67 and 70 of the light chain defined by the Chothia method; and
- amino acid residues at positions 63, 65, 67, 70 and 72 of the light chain defined by the Chothia method.

Hereinafter, for variants of each antibody, a variant in which amino acid residues at positions 63, 65 and 67 of the light chain defined by the Chothia method are changed to arginine residues is called "R3 variant", a variant in which amino acid residues at positions 63, 65, 67 and 70 of the light chain defined by the Chothia method are changed to arginine residues is called "R4 variant", and a variant in which amino acid residues at positions 63, 65, 67, 70 and 72 of the light chain defined by the Chothia method are changed to arginine residues is called "R5 variant". For the anti-IL-12 antibody and the anti-IL-4 antibody, R5 variants were not prepared.

Using each of the acquired plasmid DNAs as a template, a PCR reaction solution with the following composition was prepared.

[PCR Reaction Solution]

| | |
|---|---|
| PrimeSTAR (registered trademark) Max DNA Polymerase | 12.5μL |
| Forward primer (10 μM) | 0.5μL |
| Reverse primer (10 μM) | 0.5μL |
| Template plasmid (1 ng/μL) | 1 μL |
| Purified water | 10.5 μL |
| Total | 25 μL |

The prepared PCR reaction solution was subjected to a PCR reaction under the following reaction conditions.

[Reaction Conditions]

10 seconds at 98°C,
25 cycles of 98°C for 10 seconds, 55°C for 5 seconds and 72°C for 40 seconds, and
72°C for 3 minutes.

The obtained PCR product was fragmented by adding 1 μL of DpnI (10 U/μL) to the PCR product (25 μL). Using the DpnI-treated PCR product, a ligation reaction solution having the following composition was prepared. The reaction solution was incubated at 16°C for 1 hour to perform a ligation reaction.

[Ligation Reaction Liquid]

| | |
|---|---|
| DpnI-treated PCR product | 2 μL |
| Ligation high ver.2 | 5 μL |
| T4 Polynucleotide kinase | 1 μL |
| Purified water | 7 μL |
| Total | 15 μL |

(2.2) Transformation, plasmid extraction and sequence confirmation

The solution (3 μL) after the ligation reaction was added to DH5α (30 μL), and the mixture was allowed to stand on ice for 30 minutes. Thereafter, the mixture was heat shocked by heating at 42°C for 45 seconds. The mixture was again allowed to stand on ice for 2 minutes, then the whole amount was applied to an ampicillin-containing LB agar medium. The agar medium was incubated at 37°C for 16 hours to obtain E. coli transformants. Single colonies on the agar medium were placed in the ampicillin-containing LB liquid medium (2 mL), and the medium was shake-cultured at 37°C for 16 hours. Plasmid DNAs were extracted from the obtained E. coli using the QIAprep Spin Miniprep kit. Confirmation of each of the obtained plasmid DNAs by sequencing was entrusted to Eurofins Genomics K.K.

(2.4) Amino acid sequence of variant

The amino acid sequences of the heavy chain and light chain of each variant (Fab) were determined based on the base sequences of the genes of the anti-IL-6 antibody, the anti-IL-8 antibody and the anti-IL-12 antibody. Also, the amino acid sequences of the heavy chain and light chain of each variant (IgG) were determined based on the base sequence of the gene of the anti-IL-4 antibody. The amino acid sequences of the light chains of variants are shown below. Underlined portion indicates an amino acid residue changed from the amino acid sequence of the original antibody.

· Amino acid sequence of light chain of R3 variant of anti-IL-6 antibody (clone 58-1)

DIVLTQSPASLAVSLGQRATISCRASESVDGFGISFMNWFQQKPGQPPK
LLIYVASNQGSGVPARF<u>R</u>G<u>R</u>G<u>R</u>GTDFS<u>L</u>NIHPMEEDDSAMYFCQQSKEVPWTF
GGGTKLEIKR  (SEQ ID NO: 43)

· Amino acid sequence of light chain of R4 variant of anti-IL-6 antibody (clone 58-1)

DIVLTQSPASLAVSLGQRATISCRASESVDGFGISFMNWFQQKPGQPPK
LLIYVASNQGSGVPARF<u>R</u>G<u>R</u>G<u>R</u>GT<u>R</u>FSLNIHPMEEDDSAMYFCQQSKEVPWTF

GGGTKLEIKR  (SEQ ID NO: 44)

· Amino acid sequence of light chain of R5 variant of anti-IL-6 antibody (clone 58-1)

DIVLTQSPASLAVSLGQRATISCRASESVDGFGISFMNWFQQKPGQPPK
LLIYVASNQGSGVPARF<u>R</u>G<u>R</u>G<u>R</u>GT<u>R</u>F<u>R</u>LNIHPMEEDDSAMYFCQQSKEVPWTF
GGGTKLEIKR  (SEQ ID NO: 45)

· Amino acid sequence of light chain of R3 variant of anti-IL-6 antibody (clone 44-15)

DIQMTQSPASLSASVGETVTITCRSSENIYSFLTWFQQRQGKSPHLLVY
YANTLAEGVPSRF<u>R</u>G<u>R</u>G<u>R</u>GTQFSLQINSLQPEDFGTYYCQHHYGTPWTFGGGT
RLEIKR          (SEQ ID NO: 46)

· Amino acid sequence of light chain of R4 variant of anti-IL-6 antibody (clone 44-15)

DIQMTQSPASLSASVGETVTITCRSSENIYSFLTWFQQRQGKSPHLLVY
YANTLAEGVPSRF<u>R</u>G<u>R</u>G<u>R</u>GT<u>R</u>FSLQINSLQPEDFGTYYCQHHYGTPWTFGGGT
RLEIKR          (SEQ ID NO: 47)

· Amino acid sequence of light chain of R5 variant of anti-IL-6 antibody (clone 44-15)

DIQMTQSPASLSASVGETVTITCRSSENIYSFLTWFQQRQGKSPHLLVY
YANTLAEGVPSRF<u>R</u>GR<u>R</u>GR<u>R</u>GT<u>R</u>F<u>R</u>LQINSLQPEDFGTYYCQHHYGTPWTFGGGT
RLEIKR          (SEQ ID NO: 48)

· Amino acid sequence of light chain of R3 variant of anti-IL-8 antibody (clone 166-1)

DIVLTQSPPSLAVSLGQRATISCKASQSVDYDGDSYMNWYQQKPGQPP
KVLIYGASNLESGIPARF<u>R</u>GR<u>R</u>GR<u>R</u>GTDFTLNIYPVEEEDAATYYCQQSNEDPPTFG
GGTKLEIKR     (SEQ ID NO: 49)

· Amino acid sequence of light chain of R4 variant of anti-IL-8 antibody (clone 166-1)

DIVLTQSPPSLAVSLGQRATISCKASQSVDYDGDSYMNWYQQKPGQPP
KVLIYGASNLESGIPARF<u>R</u>GR<u>R</u>GR<u>R</u>GT<u>R</u>FTLNIYPVEEEDAATYYCQQSNEDPPTFG
GGTKLEIKR     (SEQ ID NO: 50)

· Amino acid sequence of light chain of R5 variant of anti-IL-8 antibody (clone 166-1)

DIVLTQSPPSLAVSLGQRATISCKASQSVDYDGDSYMNWYQQKPGQPP
KVLIYGASNLESGIPARF<u>R</u>GR<u>R</u>GR<u>R</u>GT<u>R</u>F<u>R</u>LNIYPVEEEDAATYYCQQSNEDPPTFG
GGTKLEIKR     (SEQ ID NO: 51)

· Amino acid sequence of light chain of R3 variant of anti-IL-8 antibody (clone 146-1)

DIQMTQSPASLSASVGETVTITCRASENIYSYLAWYQQKQGKSPHLLVY
DAETLAEGVPSRF<u>R</u>GR<u>R</u>GR<u>R</u>GTQFSLKINSLQPEDFGSYYCQHHYGTPWTFGGGT
KLEIKR          (SEQ ID NO: 52)

· Amino acid sequence of light chain of R4 variant of anti-IL-8 antibody (clone 146-1)

DIQMTQSPASLSASVGETVTITCRASENIYSYLAWYQQKQGKSPHLLVY
DAETLAEGVPSRF<u>R</u>GR<u>R</u>GR<u>R</u>GT<u>R</u>FSLKINSLQPEDFGSYYCQHHYGTPWTFGGGT
KLEIKR          (SEQ ID NO: 53)

· Amino acid sequence of light chain of R5 variant of anti-IL-8 antibody (clone 146-1)

DIQMTQSPASLSASVGETVTITCRASENIYSYLAWYQQKQGKSPHLLVY DAETLAEGVPSRFRGRGRGTRFRLKINSLQPEDFGSYYCQHHYGTPWTFGGGT KLEIKR        (SEQ ID NO: 54)

· Amino acid sequence of light chain of R3 variant of anti-IL-12 antibody (clone 22-4)

DIVMTQSHKFMSTSVGDRVSITCKASQDVGTAVAWYQQKPGQSPKLLI YWASTRHTGVPGRFRGRGRGTDFTLTINNVQSEDLADYFCQQYSSYPLTFGAG TKLELKR        (SEQ ID NO: 55)

· Amino acid sequence of light chain of R4 variant of anti-IL-12 antibody (clone 22-4)

DIVMTQSHKFMSTSVGDRVSITCKASQDVGTAVAWYQQKPGQSPKLLI YWASTRHTGVPGRFRGRGRGTRFTLTINNVQSEDLADYFCQQYSSYPLTFGAG TKLELKR        (SEQ ID NO: 56)

· Amino acid sequence of light chain of R5 variant of anti-IL-12 antibody (clone 22-4)

DIVMTQSHKFMSTSVGDRVSITCKASQDVGTAVAWYQQKPGQSPKLLI YWASTRHTGVPGRFRGRGRGTRFRLTINNVQSEDLADYFCQQYSSYPLTFGAG

TKLELKR        (SEQ ID NO: 57)

· Amino acid sequence of light chain of R3 variant of anti-IL-4 antibody

DIQMTQSPASLSVSVGDTITLTCHASQNIDVWLSWFQQKPGNIPKLLIY KASNLHTGVPSRFRGRGRGTGFTLTISSLQPEDIATYYCQQAHSYPFTFGGGTKL EIKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTMSFNRG EC        (SEQ ID NO: 58)

· Amino acid sequence of light chain of R4 variant of anti-IL-4 antibody

DIQMTQSPASLSVSVGDTITLTCHASQNIDVWLSWFQQKPGNIPKLLIY
KASNLHTGVPSRFRGRGRGTRFTLTISSLQPEDIATYYCQQAHSYPFTFGGGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTMSFNRG
EC      (SEQ ID NO: 59)

(3) Expression of wild-type antibodies and variants in mammalian cells

Hereinafter, the prepared plasmid DNAs were used as plasmid DNAs for expressing mammalian cells.

[Reagents]

Expi293 (trademark) cells (Invitrogen)
Expi293 (trademark) Expression medium (Invitrogen)
ExpiFectamine (trademark) 293 transfection kit (Invitrogen)

(3.1) Transfection

Expi293 Cells were proliferated by shaking culture (125 rpm) at 37°C in a 5% $CO_2$ atmosphere. 30 mL of cell culture ($3 \times 10^6$ cells/mL) was prepared according to the number of samples. A DNA solution with the following composition was prepared using a plasmid DNA encoding a heavy chain and a light chain of wild-type antibodies and variants thereof. The DNA solution was allowed to stand for 5 minutes.

[DNA Solution]

| | |
|---|---|
| Light chain plasmid solution | Amount ($\mu$L) corresponding to 15 $\mu$g |
| Heavy chain plasmid solution | Amount ($\mu$L) corresponding to 15 $\mu$g |
| Opti-MEM (trademark) | Appropriate amount (mL) |
| Total | 1.5 mL |

A transfection reagent having the following composition was prepared. The transfection reagent was allowed to stand for 5 minutes.

| | |
|---|---|
| ExpiFectamine reagent | 80 $\mu$L |
| Opti-MEM (trademark) | 1420 $\mu$L |
| Total | 1.5 mL |

The prepared DNA solution and the transfection reagent were mixed. The mixture was allowed to stand for 20 minutes. The resulting mixture (3 mL) was added to the cell culture (30 mL). The mixture was shake-cultured (125 rpm) at 37°C for 20 hours in a 5% $CO_2$ atmosphere. After 20 hours, 150 $\mu$L and 1.5 mL of ExpiFectamine (trademark) transfection enhancers 1 and 2 were added to each culture, respectively. Each mixture was shake-cultured (125 rpm) at 37°C for 4 days in a 5% $CO_2$ atmosphere.

(3.2) Recovery and purification of antibody

Each cell culture was centrifuged at 3,000 rpm for 15 minutes, and the culture supernatant was recovered. The culture supernatant contains each antibody secreted from transfected Expi293 (trademark) cells. The obtained culture supernatant was again centrifuged at $10,000 \times G$ for 10 minutes, and the supernatant was recovered. 300 $\mu$L of a carrier for antibody purification HisPur Ni-NTA Superflow (Thermo Fisher Scientific) was added to the obtained supernatant (30 mL), and the mixture was reacted at 4°C for 16 hours. The carrier was recovered to remove the supernatant, and TBS (1 mL) was added to wash the carrier. To the carrier was added 1000 $\mu$L of TBS containing 300 mM imidazole to elute the antibody captured on the carrier. This elution operation was performed a total of 3 times to obtain an antibody solution.

(4) Measurement of affinity for antigen

(4.1) For anti-IL-6 antibody, anti-IL-8 antibody, and anti-IL-12 antibody

Affinity of the original antibodies and variants was measured using Biacore (registered trademark) 8K (Cytiva). As antigens, human IL6 protein, His and GST tags (active) (GTX00094-pro: GeneTex, Inc.), interleukin 8 (CXCL8) (100-175: Shenandoah Biotechnology, Inc.), and human IL 12 protein (Recombinant His, C-terminus) (aa23-328) (LS-G134297-50: LSBio, Inc.) were used. The antigen was immobilized to a sensor chip for Biacore (registered trademark) Series S Sensor Chip CM4 (GE Healthcare). Each antibody solution obtained in the above (3.2) was diluted to prepare an antibody solution for measurement. The concentration of each antibody in the antibody solution for measurement was as follows. In the solutions of the anti-IL-6 antibody (58-1) and its R3, R4 and R5 variants, the concentration was 100 nM, 50 nM, 25 nM, 12.5 nM and 6.25 nM. In the solution of the anti-IL-6 antibody (44-15), the concentration was 100 nM, 50 nM, 25 nM, 12.5 nM and 6.25 nM, and in the solutions of its R3, R4 and R5 variants, the concentration was 50 nM, 25 nM, 12.5 nM, 6.25 nM and 3.13 nM. In the solutions of the anti-IL-8 antibody (166-1) and its R3, R4 and R5 variants, the concentration was 100 nM, 50 nM, 25 nM, 12.5 nM and 6.25 nM. In the anti-IL-8 antibody (146-1) solution, the concentration was 100 nM, 50 nM, 25 nM, 12.5 nM and 6.25 nM, in the solution of its R3 variant, the concentration was 50 nM, 25 nM, 12.5 nM, 6.25 nM and 3.13 nM, and in the solutions of its R4 and R5 variants, the concentration was 25 nM, 12.5 nM, 6.25 nM, 3.13 nM and 1.56 nM. In the anti-IL-12 antibody solution, the concentration was 50 nM, 25 nM, 12.5 nM, 6.25 nM and 3.13 nM, in the solution of its R3 variant, the concentration was 25 nM, 12.5 nM, 6.25 nM, 3.13 nM and 1.56 nM, and in the solution of its R4 variant, the concentration was 12.5 nM, 6.25 nM, 3.13 nM, 1.56 nM and 0.78 nM.
The antibody solutions for measurement at each concentration were delivered to Biacore (registered trademark) 8K (Cytiva) (association time of 120 seconds and dissociation time of 1200 seconds). Measurement data was analyzed using Biacore (registered trademark) Evaluation software, and the data on the affinity of each antibody for an antigen was obtained.

(4.2) For anti-IL-4 antibody

Affinity of the original anti-IL-4 antibody and variants was measured by ELISA method. As an antigen, an animal free recombinant human IL-4 (AF-200-04: Peprotech, Inc.) was used. The IL-4 was diluted with 1% BSA/TBS to prepare an antigen solution (5 $\mu$g/mL). Each of the original anti-IL-4 antibody, R3 variant and R4 variant was diluted with 1% BSA/TBS to prepare an antibody solution (1000 ng/mL, 250 ng/mL, 62.5 ng/mL, 15.63 ng/mL, 3.91 ng/mL, 0.98 ng/mL, and 0.244 ng/mL). As a detection antibody, an HRP-labeled anti-human IgG antibody (Bethyl Laboratories) was used. The detection antibody was diluted with 1% BSA/TBS to prepare a detection antibody solution (100 ng/mL).
The antigen solution was dispensed at 50 $\mu$L per well into a 96 well plate for ELISA (Thermo Fisher Scientific), and the plate was incubated at 4°C overnight. The plate was washed 3 times with 200 $\mu$L of a washing solution per well (0.05% Tween 20-containing TBS). A blocking solution (1% BSA-containing TBS) was dispensed at 250 $\mu$L per well, and the plate was incubated at 4°C overnight. The blocking solution was removed, the antigen solution was dispensed at 50 $\mu$L of antibody solution per well into the plate, and the plate was incubated at room temperature for 15 minutes. After washing the plate 3 times with a washing solution, the detection antibody solution was dispensed at 50 $\mu$L per well, and the plate was incubated at room temperature for 1 hour. After washing the plate 3 times with a washing solution, a substrate solution (Seracare) was dispensed at 100 $\mu$L per well into the plate, and the absorbance at 450 nm was measured with a plate reader SpectraMax 190 Microplate Reader (Molecular Devices, LLC.).

(5) Results

$K_D$ values of the original anti-IL-6 antibody, anti-IL-8 antibody and anti-IL-12 antibody (unmodified) and variants thereof are shown in Tables 7 to 11. Also, based on the $K_D$ value, the affinity ratios of the variants when the affinity of the original antibody was 1.0 ($K_D$ ratios) are also shown. The measurement results of the ELISA method of the original anti-IL-4 antibody (unmodified) and variants thereof are shown in Fig. 1. The values of EC50 for the original anti-IL-4 antibody (unmodified) and the variants are shown in Table 12. Also, based on the value of EC50, the affinity ratios of the variants when the affinity of the original anti-IL-4 antibody was (EC50 ratio) 1.0 are also shown.

[Table 7]

| Anti-IL-6 antibody (clone 58-1) | | |
|---|---|---|
| **Sample** | **$K_D$ (M)** | **$K_D$ ratio** |
| Unmodified | 1.98E-10 | 1.0 |
| **R3** | 8.12E-11 | 2.4 |
| **R4** | 4.72E-11 | 4.2 |
| **R5** | 2.79E-11 | 1.1 |

[Table 8]

| Anti-IL-6 antibody (clone 44-15) | | |
|---|---|---|
| **Sample** | **$K_D$ (M)** | **$K_D$ ratio** |
| Unmodified | 2.04E-08 | 1.0 |
| **R3** | 7.16E-09 | 2.9 |
| **R4** | 8.38E-09 | 2.4 |
| **R5** | 4.40E-09 | 4.6 |

[Table 9]

| Anti-IL-8 antibody (clone 166-1) | | |
|---|---|---|
| **Sample** | **$K_D$ (M)** | **$K_D$ ratio** |
| Unmodified | 1.64E-09 | 1.0 |
| **R3** | 1.17E-09 | 1.4 |
| **R4** | 5.95E-10 | 2.8 |
| **R5** | 3.89E-10 | 4.2 |

[Table 10]

| Anti-IL-8 antibody (clone 146-1) | | |
|---|---|---|
| **Sample** | $K_D$ (M) | $K_D$ ratio |
| Unmodified | 1.53E-08 | 1.0 |
| **R3** | 5.80E-09 | 2.6 |
| **R4** | 3.14E-09 | 4.9 |
| **R5** | 2.40E-09 | 6.4 |

[Table 11]

| Anti-IL-12 antibody (clone 22-4) | | |
|---|---|---|
| **Sample** | $K_D$ (M) | $K_D$ ratio |
| Unmodified | 1.03E-08 | 1.0 |
| **R3** | 4.97E-10 | 20.8 |
| **R4** | 2.45E-10 | 42.2 |

[Table 12]

| Anti-IL-4 antibody | | |
|---|---|---|
| **Sample** | EC50 (ng/mL) | EC50 ratio |
| Unmodified | 304.1 | 1.0 |
| **R3** | 179.8 | 1.7 |
| **R4** | 176.1 | 1.7 |

As shown in Tables 7 to 11, the $K_D$ values of all variants of the anti-IL-6 antibody, the anti-IL-8 antibody and the anti-IL-12 antibody were lower than the $K_D$ value of the original antibody. Also, as shown in Table 12 and Fig. 1, the value of EC50 of the variant of the anti-IL-4 antibody was lower than the value of EC50 of the original antibody. As described above, the variants of the anti-IL-6 antibody, the anti-IL-8 antibody, the anti-IL-12 antibody and the anti-IL-4 antibody had improved affinity for an antigen as compared with the respective original antibodies.

**Claims**

1.   A method for improving an affinity of an anti-cytokine antibody for an antigen, the method comprising:

in an anti-cytokine antibody whose electrical characteristic of complementarity determining regions (CDRs) is negatively charged, changing at least 3 amino acid residues of light chain framework region 3 to arginine residues or lysine residues, thereby improving an affinity for an antigen as compared with that of an antibody before the at least 3 amino acid residues are changed to arginine residues or lysine residues,
wherein the electrical characteristic of the CDRs is determined by a following formula:

$$X = [\text{Number of basic amino acid residues in amino acid sequences of the CDRs contained in one antigen binding site}] - [\text{Number of acidic amino acid residues in amino acid sequences of the CDRs contained in one antigen binding site}]$$

wherein when X is -2 or less, the electrical characteristic of the CDRs is negatively charged,
when X is -1, 0, or 1, the electrical characteristic of the CDRs is neutral, and
when X is 2 or more, the electrical characteristic of the CDRs is positively charged, and
the at least 3 amino acid residues comprise residues at least 3 positions selected from the group consisting of positions 63, 65, 67, 70 and 72 of a light chain defined by Chothia method.

2.   A method for producing an anti-cytokine antibody with an improved affinity for an antigen, the method comprising:

in an anti-cytokine antibody whose electrical characteristic of complementarity determining region (CDRs) is negatively charged, generating an antibody in which at least 3 amino acid residues of light chain framework region 3 are changed to arginine residues or lysine residues; and
recovering the antibody generated in the generating,

wherein an affinity of the recovered antibody for an antigen is improved as compared with that of an antibody before the at least 3 amino acid residues are changed to arginine residues or lysine residues,
the electrical characteristic of the CDRs is determined by a following formula:

$$X = \text{[Number of basic amino acid residues in amino acid sequences of the CDRs contained in one antigen binding site]} - \text{[Number of acidic amino acid residues in amino acid sequences of the CDRs contained in one antigen binding site]}$$

wherein when X is -2 or less, the electrical characteristic of the CDRs is negatively charged,
when X is -1, 0, or 1, the electrical characteristic of the CDRs is neutral, and
when X is 2 or more, the electrical characteristic of the CDRs is positively charged, and
the at least 3 amino acid residues comprise residues at least 3 positions selected from the group consisting of positions 63, 65, 67, 70 and 72 of a light chain defined by Chothia method.

3. The method according to claim 1 or 2, wherein the anti-cytokine antibody is an anti-interleukin antibody.

4. The method according to claim 3, wherein the anti-interleukin antibody is an anti-IL-6 antibody, an anti-IL-8 antibody, an anti-IL-12 antibody, or an anti-IL-4 antibody.

5. The method according to any one of claims 1 to 4, wherein the at least 3 amino acid residues comprise amino acid residues at positions 63, 65 and 67 of the light chain.

6. The method according to any one of claims 1 to 5, wherein the at least 3 amino acid residues comprise amino acid residues at positions 63, 65, 67 and 70 of the light chain.

7. The method according to any one of claims 1 to 6, wherein the at least 3 amino acid residues comprise amino acid residues at positions 63, 65, 67, 70 and 72 of the light chain.

8. An anti-cytokine antibody in which at least 3 amino acid residues of light chain framework region 3 are changed to arginine residues or lysine residues, wherein the anti-cytokine antibody is an antibody whose electrical characteristic of complementarity determining region (CDRs) is negatively charged, and the electrical characteristic of the CDRs is determined by a following formula:

$$X = \text{[Number of basic amino acid residues in amino acid sequences of the CDRs contained in one antigen binding site]} - \text{[Number of acidic amino acid residues in amino acid sequences of the CDRs contained in one antigen binding site]}$$

wherein when X is -2 or less, the electrical characteristic of the CDRs is negatively charged,
when X is -1, 0, or 1, the electrical characteristic of the CDRs is neutral, and
when X is 2 or more, the electrical characteristic of the CDRs is positively charged, and
the at least 3 amino acid residues comprise residues at least 3 positions selected from the group consisting of positions 63, 65, 67, 70 and 72 of a light chain defined by Chothia method, and an affinity for an antigen is higher than that of an antibody before the at least 3 amino acid residues are changed to arginine residues or lysine residues.

9. The antibody according to claim 8, wherein the anti-cytokine antibody is an anti-interleukin antibody.

10. The antibody according to claim 9, wherein the anti-interleukin antibody is an anti-IL-6 antibody, an anti-IL-8 antibody, an anti-IL-12 antibody, or an anti-IL-4 antibody.

11. The antibody according to any one of claims 8 to 10, wherein the at least 3 amino acid residues comprise amino acid residues at positions 63, 65 and 67 of the light chain.

**12.** The antibody according to any one of claims 8 to 10, wherein the at least 3 amino acid residues comprise amino acid residues at positions 63, 65, 67 and 70 of the light chain.

**13.** The antibody according to any one of claims 8 to 10, wherein the at least 3 amino acid residues comprise amino acid residues at positions 63, 65, 67, 70 and 72 of the light chain.

# *FIGURE*

Europäisches
Patentamt

European
Patent Office

Office européen
des brevets

## EUROPEAN SEARCH REPORT

**Application Number**

EP 22 19 5750

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 342 783 A1 (SYSMEX CORP [JP]) 4 July 2018 (2018-07-04) * paragraph 0007,0012,0028; claims 1,5,6,7,10,14; example 1 * | 1-13 | INV. C07K16/24 |
| A | FUKUNAGA ATSUSHI ET AL: "Improvement of antibody affinity by introduction of basic amino acid residues into the framework region", BIOCHEMISTRY AND BIOPHYSICS REPORTS, vol. 15, 1 September 2018 (2018-09-01), pages 81-85, XP055828390, ISSN: 2405-5808, DOI: 10.1016/j.bbrep.2018.07.005 * page 84; figure 1 * | 1-13 | |
| A | EP 3 854 808 A1 (SYSMEX CORP [JP]) 28 July 2021 (2021-07-28) * claim 1 * | 1-13 | |
| A | A. FUKUNAGA ET AL: "Improving the affinity of an antibody for its antigen via long-range electrostatic interactions", PROTEIN ENGINEERING, DESIGN AND SELECTION, vol. 26, no. 12, 7 November 2013 (2013-11-07), pages 773-780, XP055473804, GB ISSN: 1741-0126, DOI: 10.1093/protein/gzt053 * abstract; page 775 * | 1-13 | **TECHNICAL FIELDS SEARCHED (IPC)** C07K |

The present search report has been drawn up for all claims

| Place of search | Date of completion of the search | Examiner |
|---|---|---|
| Munich | 30 January 2023 | Mattugini, Nicola |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 19 5750

30-01-2023

| Patent document cited in search report | | | Publication date | Patent family member(s) | | | Publication date |
|---|---|---|---|---|---|---|---|
| EP 3342783 | A1 | | 04-07-2018 | CN | 108250294 | A | 06-07-2018 |
| | | | | EP | 3342783 | A1 | 04-07-2018 |
| | | | | US | 2018179298 | A1 | 28-06-2018 |
| | | | | US | 2022235147 | A1 | 28-07-2022 |
| EP 3854808 | A1 | | 28-07-2021 | CN | 113248600 | A | 13-08-2021 |
| | | | | CN | 113248601 | A | 13-08-2021 |
| | | | | CN | 113248602 | A | 13-08-2021 |
| | | | | CN | 113248603 | A | 13-08-2021 |
| | | | | CN | 113248604 | A | 13-08-2021 |
| | | | | CN | 113248605 | A | 13-08-2021 |
| | | | | CN | 114502581 | A | 13-05-2022 |
| | | | | CN | 114555642 | A | 27-05-2022 |
| | | | | EP | 3854807 | A1 | 28-07-2021 |
| | | | | EP | 3854808 | A1 | 28-07-2021 |
| | | | | EP | 3854809 | A1 | 28-07-2021 |
| | | | | EP | 3854810 | A1 | 28-07-2021 |
| | | | | EP | 3854811 | A1 | 28-07-2021 |
| | | | | EP | 3854812 | A1 | 28-07-2021 |
| | | | | EP | 4095154 | A1 | 30-11-2022 |
| | | | | EP | 4095155 | A1 | 30-11-2022 |
| | | | | JP | WO2021149427 | A1 | 29-07-2021 |
| | | | | JP | WO2021149428 | A1 | 29-07-2021 |
| | | | | TW | 202128748 | A | 01-08-2021 |
| | | | | TW | 202128766 | A | 01-08-2021 |
| | | | | US | 2021230304 | A1 | 29-07-2021 |
| | | | | US | 2021230305 | A1 | 29-07-2021 |
| | | | | US | 2021230306 | A1 | 29-07-2021 |
| | | | | US | 2021230307 | A1 | 29-07-2021 |
| | | | | US | 2021261687 | A1 | 26-08-2021 |
| | | | | US | 2022220191 | A1 | 14-07-2022 |
| | | | | US | 2022230705 | A1 | 21-07-2022 |
| | | | | WO | 2021149427 | A1 | 29-07-2021 |
| | | | | WO | 2021149428 | A1 | 29-07-2021 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180179298

- WO 2013084371 A

**Non-patent literature cited in the description**

- **CHOTHIA C. ; LESK AM.** Canonical Structures for the Hypervariable Regions of Immunoglobulins. *J Mol Biol.*, 1987, vol. 196, 901-917

- **KOHLER ; MILSTEIN.** *Nature,* 1975, vol. 256, 495-497
- **FUKUNAGA A. ; TSUMOTO K.** *Protein Eng. Des. Sel.,* 2013, vol. 26, 773-780